# EUROPEAN PATENT SPECIFICATION

(11) **EP 0 932 394 B1**
(45) Date of publication and mention of the grant of the patent: **04.06.2003**
(21) Application number: 97945023.6
(22) Date of filing: 17.10.1997
(51) Int. Cl.: A61K 9/20, A23L 1/302

(54) **VITAMIN PRODUCT**
VITAMINPRODUKT
PRODUIT DE VITAMINES

(30) Priority: 17.10.1996 GB 9621614
(43) Date of publication of application: 04.08.1999
(73) Proprietor: Cultech Limited, Swansea, West Glamorgan SA1 3NJ (GB)
(72) Inventor: PLUMMER, Nigel, Terrence, Swansea, West Glamorgan SA6 6AQ (GB)
(74) Representative: Swindell & Pearson
(86) International application number: GB9702863
(87) International publication number: WO98017258

(56) References cited:
- EP-A- 0 450 141
- WO-A-86/01686
- WO-A-93/23017
- DATABASE WPI Section Ch, Week 9148 Derwent Publications Ltd., London, GB; Class B07, AN 91-351422 XP002056728 & SU 1 616 576 A (DAIRY RAW MALLS COMPLX U) , 30 December 1990

## Description

The present invention relates to vitamin delivery and particularly, but not exclusively, to the preparation of vitamins for delivery to subjects on a voluntary basis without medical prescription.

It is well recognised that various deficiency disorders can arise if vitamin intake is not adequate and for this reason, the health authorities in many countries specify recommended daily allowances (RDAs) of various vitamins for children and adults. Vitamins can be delivered to the subject in a number of ways. The most common form is for a discrete dosage to be ingested in tablet form or by hard or soft gelatine capsules, or by means of powders or liquids. Another common technique of delivering vitamins is by "fortifying" food or beverage products with additional prophylactic components such as vitamins. These food and beverage products may be soft drinks, cereals, dairy products, fruit juices or confectionery.

Capsules, tablets and other discrete dosage forms have the disadvantage that bulking agents (or excipients) are usually required in order to produce a tablet which is sufficiently large for convenient handling and storage. In consequence, the subject taking the capsule or tablet will ingest a considerable amount of bulking agent (which is wholly unnecessary to the prophylactic purpose) along with the desired vitamin content.

For marketing to children in particular, various fruit flavourings, natural colours and fruit powders have been used to produce tablets which are more palatable and therefore more likely to be used with adequate regularity, but considerable volumes of excipient are required.

The present invention seeks to improve vitamin delivery.

The invention provides a method of preparing a vitamin for delivery to a subject, in which at least one vitamin is added to a wet carrier to form a mix which is subsequently freeze-dried, the freeze-dried mix being ingestible.

The term "wet" is used to indicate a form which incorporates more than a negligible amount of water, e.g. a form in which water can be extracted by a freeze-drying process. Preferably the vitamin or vitamins are in solubilised form. Some vitamins are soluble in water; others require treatment to allow emulsification for dispersal in water. Vitamin mixtures are commercially available which contain soluble vitamins and treated insoluble vitamins. The term solubilised is used herein to encompass soluble vitamins, treated insoluble vitamins and mixtures of these.

The wet carrier is preferably a natural material and may be formed from fruit. The wet carrier may comprise fruit juice and/or fruit pulp.

A blend of vitamins may be added as aforesaid. The amount of vitamin added to the carrier may be calculated by reference to the vitamin content or concentration required in the freeze-dried product.

Preferably the mix is freeze-dried in discrete units. The vitamin concentration in the mix is preferably chosen to cause each freeze-dried unit to incorporate a predetermined dose of vitamin. The predetermined dose may be a recommended daily allowance or simple fraction thereof.

The invention also provides a vitamin delivery product comprising an ingestible freeze-dried mix of at least one vitamin and a carrier.

The carrier is preferably a natural material and may be formed from fruit. The carrier may be formed from fruit juice and/or fruit pulp.

Preferably the product comprises a blend of vitamins. The mix is preferably freeze-dried in discrete units, with each unit preferably incorporating a predetermined dose of vitamin, such as a recommended daily allowance.

The present invention will now be described in more detail, by way of example only, and with reference to the accompanying drawings, in which:-
Fig. 1 is a highly schematic diagram illustrating a first stage of a method according to the invention; and
Fig. 2 illustrates a later stage of the method of Fig. 1.

The method to be described is for preparing a vitamin for delivery to a subject, such as a human, and is particularly applicable to vitamin supplements for delivery on a voluntary basis, without medical supervision, such as vitamin delivery products intended to provide a recommended daily allowance of one or more vitamins.

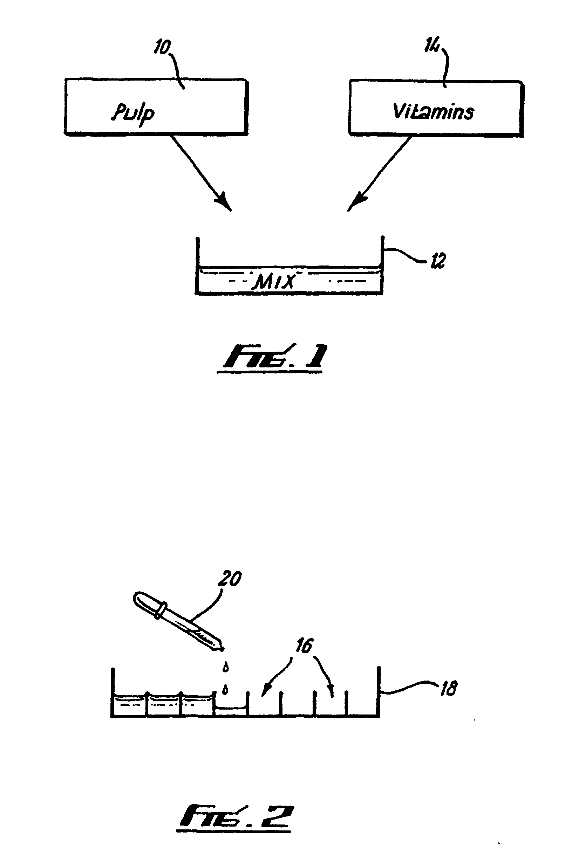

In order to produce the product, a wet carrier is first produced (indicated at 10 in Fig. 1) and introduced into a mixing vessel 12. The wet carrier is preferably a natural material which may be formed from fruit. It is envisaged that fresh or frozen fruit can be formed into a puree or (by removal of fruit pulp) juice to serve as the wet carrier. Typical fruit pulps will have a dry solids content of approximately 10% to 13%, while typical fruit juice will have a dry solids content of approximately 5% to 10%. Concentrates with higher solids contents could be used The wet carrier 10 is introduced into the mixing vessel 12.

A vitamin or mixture of vitamins is then formed (indicated at 14 in Fig.. 1) in a solubilised form and added to the wet carrier already in the mixing vessel 12. Naturally the sequence can be reversed, but it is envisaged that the volume of carrier will exceed the volume of vitamin material and thus that mixing will be facilitated by the introduction of vitamin into the carrier, rather than vice versa.

The contents of the vessel 12 are then mixed to a homogeneous mixture, with agitation or other mixing technique. The solubilised form of the vitamin content enhances the homogeneity of the mixture.

The homogeneous mixture so formed is then dispensed from the vessel 12 into individual cavities 16 of a mould tray 18 (Fig. 2). This task of dispensing is illustrated schematically by means of a pipette 20 but any alternative arrangement appropriate to the nature of the material and volumes to be dispensed could be used.

The contents of the tray 18 are then freeze-dried to substantially remove the water content thereof. Since freeze-drying involves minimal shrinkage of the dry solids content of the mix, the result is a block of dried material in each mould 16 and having the shape and size of the packet of liquid originally introduced into the mould 16. These dried units can then be removed from the moulds and packaged in an appropriate manner, such as in blister packs for retail sale as a vitamin supplement.

The vitamin delivery product so produced will contain only the dried fruit and vitamin, without excipient. Its taste will be virtually wholly determined by the original fruit content of the carrier 10. This is expected to provide a palatability and mouth feel which are greatly improved over known vitamin supplements and similar products, so that the product described is expected to be well received in the children's market.

It is preferred that each dried unit contains a recommended daily allowance of the vitamin or vitamin blend, or a simple fraction thereof (such as a half, quarter etc.).

The concentration of vitamins required in the mix formed in the vessel 12 can be calculated as follows. First, the vitamin dose to be delivered by each unit is chosen. This might, for instance, be 100mg. Secondly, the volume of the final unit is chosen (perhaps 1ml), largely for practical reasons of handling and user preference. The mix in the vessel 12 is thus required to have a vitamin concentration of one dose per final unit volume. Other factors may influence final choices. For instance, some vitamins have stronger or more unpleasant tastes than others, requiring a higher ratio of fruit content to vitamin content to disguise the vitamin taste. The final unit volume might therefore be increased to assist in this way. The dry solids content of the fruit component also affects the final product, in that a material with a higher dry solids content is more likely to adequately mask a vitamin taste, than would a material with a lower dry solids content. These factors may affect the choice of fruit material (e.g. the fruit type) or the form (juice, fruit pulp, concentrate etc.). The mouth feel and taste are affected by these choices. A final product can be produced which consists of a honeycomb structure of dried fruit solid, through which the vitamin dose is evenly distributed, and which has an acceptable taste and texture in the mouth.

It is expected that the technique can be applied to a wide variety of natural materials, particularly a wide variety of fruit juices and pulps, and to many different vitamins and vitamin blends, so that a wide range of vitamin delivery products can be produced for many different purposes.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. A method of preparing a vitamin product for delivery to a subject, in which at least one vitamin is added to a wet carrier to form a mix which is subsequently freeze-dried, the freeze-dried mix being ingestible.

2. A method according to Claim 1, wherein the vitamin or vitamins are in solubilised form.

3. A method according to Claim 1 or 2, wherein the wet carrier is a natural material.

4. A method according to Claim 3, wherein the wet carrier is formed from fruit.

5. A method according to Claim 3 or 4, wherein the wet carrier comprises fruit juice and/or fruit pulp.

6. A method according to any preceding claim, wherein a blend of vitamins is added as aforesaid.

7. A method according to any preceding claim, wherein the amount of vitamin added to the carrier is calculated by reference to the vitamin content or concentration required in the freeze-dried product.

8. A method according to any preceding claim, wherein the mix is freeze-dried in discrete units.

9. A method according to any preceding claim wherein the vitamin concentration in the mix is chosen to cause each freeze-dried unit to incorporate a predetermined dose of vitamin.

10. A method according to claim 9, wherein the predetermined dose is a recommended daily allowance or simple fraction thereof.

11. A vitamin delivery product comprising an ingestible freeze-dried mix of at least one vitamin and a carrier.

12. A product according to claim 11, wherein the carrier is a natural material.

13. A product according to Claim 12, wherein the carrier is formed from fruit.

14. A product according to Claim 12 or 13, wherein the carrier is formed from fruit juice and/or fruit pulp.

15. A product according to any of Claims 11 to 14, comprising a blend of vitamins.

16. A product according to any of Claims 11 to 15, wherein the mix is freeze-dried in discrete units.

17. A product according to Claim 16, wherein each unit incorporates a predetermined dose of vitamin.

18. A product according to Claim 17, wherein each unit incorporates a recommended daily allowance.

## Patentansprüche

1. Verfahren zum Herstellen eines Vitaminprodukts zur Verabreichung an ein Subjekt, wobei mindestens ein Vitamin einem nassen Träger hinzugefügt wird, um eine Mischung zu bilden, die anschließend gefriergetrocknet wird, wobei die gefriergetrocknete Mischung vom Körper aufnehmbar ist.

2. Verfahren nach Anspruch 1, bei dem das Vitamin oder die Vitamine in gelöster Form vorliegen.

3. Verfahren nach Anspruch 1 oder 2, bei dem der nasse Träger ein natürliches Material ist.

4. Verfahren nach Anspruch 3, bei dem der nasse Träger aus Obst gebildet wird.

5. Verfahren nach Anspruch 3 oder 4, bei dem der nasse Träger Fruchtsaft und/oder Fruchtfleisch aufweist.

6. Verfahren nach einem der vorhergehenden Ansprüche, bei dem eine Mischung aus Vitaminen wie zuvor gesagt hinzugefügt wird.

7. Verfahren nach einem der vorhergehenden Ansprüche, bei dem die Menge des dem Träger hinzugefügten Vitamins unter Bezugnahme auf den Vitamingehalt oder die Konzentration berechnet wird, die in dem gefriergetrockneten Produkt benötigt werden.

8. Verfahren nach einem der vorhergehenden Ansprüche, bei dem die Mischung in diskreten Einheiten gefriergetrocknet wird.

9. Verfahren nach einem der vorhergehenden Ansprüche, bei die Vitaminkonzentration in der Mischung so ausgewählt wird, dass sie bewirkt, dass jede gefriergetrocknete Einheit eine vorbestimmte Vitamindosis enthält.

10. Verfahren nach Anspruch 9, bei dem die vorbestimmte Dosis die empfohlene tägliche Verabreichungsmenge oder ein einfacher Bruchteil davon ist.

11. Vitamin-Verabreichungsprodukt mit einer vom Körper aufnehmbaren gefriergetrockneten Mischung aus mindestens einem Vitamin und einem Träger.

12. Produkt nach Anspruch 11, bei dem Träger ein natürliches Material ist.

13. Produkt nach Anspruch 12, bei dem der Träger aus Obst gebildet ist.

14. Produkt nach Anspruch 12 oder 13, bei dem der Träger aus Fruchtsaft und/oder Fruchtfleisch gebildet ist.

15. Produkt nach einem der Ansprüche 11 bis 14, das ein Gemisch aus Vitaminen aufweist.

16. Produkt nach einem der Ansprüche 11 bis 15, bei dem die Mischung in diskreten Einheiten gefriergetrocknet ist.

17. Produkt nach Anspruch 16, bei dem jede Einheit eine vorbestimmte Dosis eines Vitamins enthält.

18. Produkt nach Anspruch 17, bei dem jede Dosis eine empfohlene tägliche Verabreichungsmenge enthält.

## Revendications

1. Procédé pour préparer un produit vitaminé destiné à être administré à un sujet, dans lequel au moins une vitamine est ajoutée à un support humide pour former un mélange qui est par la suite lyophilisé, le mélange lyophilisé pouvant être ingéré.

2. Procédé selon la revendication 1, dans lequel la vitamine ou les vitamines sont sous une forme solubilisée.

3. Procédé selon la revendication 1 ou 2, dans lequel le support humide est un matériau naturel.

4. Procédé selon la revendication 3, dans lequel le support humide est formé à partir d'un fruit.

5. Procédé selon la revendication 3 ou 4, dans lequel le support humide comporte du jus de fruit et/ou de la pulpe de fruit.

6. Procédé selon l'une quelconque des revendications précédentes, dans lequel une combinaison de vitamines est ajoutée comme mentionné ci-dessus.

7. Procédé selon l'une quelconque des revendications précédentes, dans laquelle la quantité de vitamines ajoutées au support est calculée en référence à la teneur en vitamines ou concentration nécessaire dans le produit lyophilisé.

8. Procédé selon l'une quelconque des revendications précédentés, dans lequel le mélange est lyophilisé en unités discrètes.

9. Procédé selon l'une quelconque des revendications précédentes, dans lequel la concentration en vitamines dans le mélange est choisie pour amener chaque unité lyophilisée à incorporer une dose prédéterminée de vitamines.

10. Procédé selon la revendication 9, dans lequel la dose prédéterminée est une tolérance quotidienne recommandée ou une fraction simple de celle-ci.

11. Produit d'apport en vitamines comportant un mélange lyophilisé pouvant être ingéré d'au moins une vitamine et d'un support.

12. Produit selon la revendication 11, dans lequel le support est un matériau naturel.

13. Produit selon la revendication 12, dans lequel le support est formé à partir d'un fruit.

14. Produit selon la revendication 12 ou 13, dans lequel le support est formé à partir de jus de fruit et/ou de pulpe de fruit.

15. Produit selon l'une quelconque des revendications 11 à 14, comportant une combinaison de vitamines.

16. Produit selon l'une quelconque des revendications 11 à 15, dans lequel le mélange est lyophilisé en unités discrètes.

17. Produit selon la revendication 16, dans lequel chaque unité comporte une dose prédéterminée de vitamines.

18. Produit selon la revendication 17, dans lequel chaque unité comporte une tolérance quotidienne recommandée.
